# EUROPEAN PATENT APPLICATION

(11) **EP 4 682 149 A1**
(43) Date of publication of application: **21.01.2026**
(21) Application number: 24774062.4
(22) Date of filing: 18.03.2024
(51) Int. Cl.: C07D 487/04, A61K 31/519, A61P 35/00

(54) **POLYMORPH OF JAK TYROSINE KINASE INHIBITOR AND USE THEREOF**

(30) Priority: 17.03.2023 CN 202310274379
(71) Applicant: Prime Gene Therapeutics Co., Ltd., Beijing 100070 (CN)
(72) Inventor: HAN, Junru, Beijing 100070 (CN); LEI, Fei, Beijing 100070 (CN); MA, Sufeng, Beijing 100070 (CN); ZHU, Li, Beijing 100070 (CN); WANG, Zhen, Beijing 100070 (CN); HOU, Fuhui, Beijing 100070 (CN)
(74) Representative: Høiberg P/S
(86) International application number: PCT/CN2024/082137
(87) International publication number: WO 2024/193497

(57) **Abstract**

The present application relates to a polymorph of 2-(3-(3-amino-4-(7H-pyrrolo[2, 3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile compound of formula (I) and a pharmaceutical use thereof.

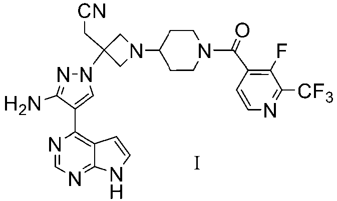

## Description

### Technical Field

The present disclosure relates to polymorphs of 2-(3-(3-amino-4-(*7H*-pyrrolo[2,3- *d*]pyrimidin-4-yl)-1H-pyrazol-1-yl)-1-(1-(3-fluoro-2-(trifluoromethyl)isonicotinyl)piperidin-4-yl)azetidin-3-yl)acetonitrile, a preparation method and medical use thereof .

### Background Art

The compound of 2-(3-(3-amino-4-(*7H*-pyrrolo[2,3-*d*]pyrimidin-4-yl)-1H-pyrazol-1-yl)-1-(1-(3-fluoro-2-(trifluoromethyl)isonicotinyl)piperidin-4-yl)azetidin-3-yl)acetonitrile belongs to JAK class of non-receptor tyrosine kinase (PTK) inhibitors. Its JAK-STAT signaling pathway is closely related to inflammatory cytokines and tumors, and is widely involved in important biological processes such as cell proliferation, differentiation, metastasis, apoptosis, regulation of immune response and cell homeostasis in human health and disease. In addition, 2-(3-(3-amino-4-(7H-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile maintains excellent JAK1 enzyme inhibition while avoiding potential side effects from inhibiting other JAK kinases (JAK2 and/or JAK3), thus possessing great application potential. The structural formula of 2-(3-(3-amino-4-(7H-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile is as follows:

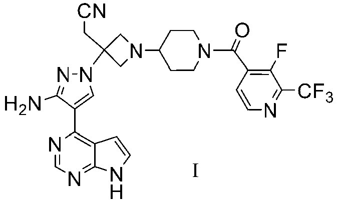

The synthesis method of this compound is disclosed in Chinese application CN109867676A, but no crystal forms of the compound is disclosed. No other literature has reported any crystal forms of 2-(3-(3-amino-4-(7H-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile, so the crystal forms need to be studied.

### Summary

The present disclosure provides crystalline forms I, II, III, IV and V of 2-(3-(3-amino-4-(7H-pyrrolo[2,3-*d*]pyrimidin-4-yl)-1H-pyrazol-1-yl)-1-(1-(3-fluoro-2-(trifluoromethyl)isonicotinyl)piperidin-4-yl)azetidin-3-yl)acetonitrile and preparation methods thereof.

In one aspect of the present disclosure, the invention provides crystalline form I of 2-(3-(3-amino-4-(*7H*-pyrrolo[2,3-*d*]pyrimidin-4-yl)-1H-pyrazol-1-yl)-1-(1-(3-fluoro-2-(trifluoromethyl)isonicotinyl)piperidin-4-yl)azetidin-3-yl)acetonitrile. The X-ray powder diffraction pattern of Form I has characteristic peaks at the following 2θ positions: 5.17, 5.55, 6.30, 8.67, 10.34, 15.96, 19.06, 19.75, 20.39, and 21.42.

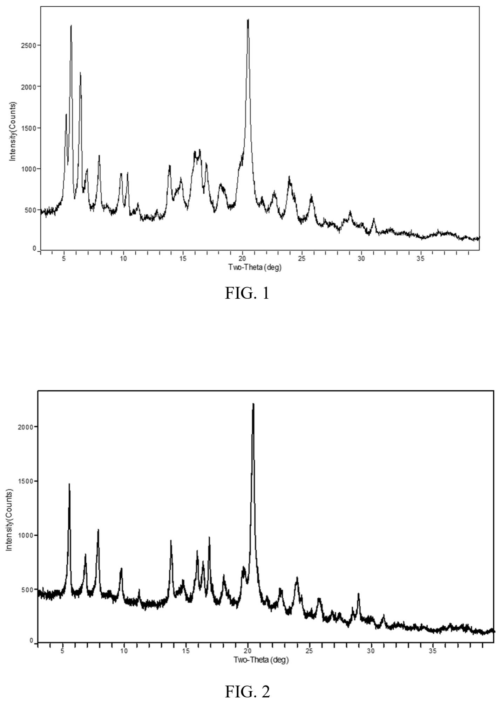

In some embodiments, the X-ray powder diffraction pattern of Form I is shown in FIG. 1.

In another aspect of the present disclosure, the invention provides crystalline form II of 2-(3-(3-amino-4-(7H-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile. The X-ray powder diffraction pattern of Form II has characteristic peaks at the following 2θ positions: 5.55, 6.85, 7.89, 9.78, 13.78, 15.89, 16.38, 16.88, 18.05, 20.42, and 24.00.

In some embodiments, the X-ray powder diffraction pattern of Form II is shown in FIG. 2.

In another aspect of the present disclosure, the invention provides crystalline form III of 2-(3-(3-amino-4-(*7H*-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile. The X-ray powder diffraction pattern of Form III has characteristic peaks at the following 2θ positions: 8.76, 9.53, 10.17, 15.11, 16.70, 19.18, 20.49, 20.79, 22.33, and 25.18.

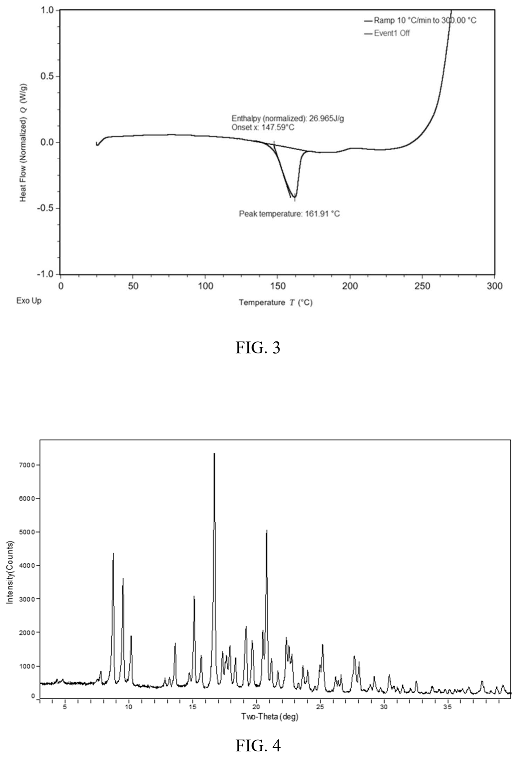

In some embodiments, the X-ray powder diffraction pattern of Form III is shown in FIG. 4.

In another aspect of the present disclosure, the invention provides crystalline form IV of 2-(3-(3-amino-4-(7H-pyrrolo[2,3-d]pyrimidin-4-yl)-1H-pyrazol-1-yl)-1-(1-(3-fluoro-2-(trifluoromethyl)isonicotinoyl)piperidin-4-yl)azetidin-3-yl)acetonitrile. The X-ray powder diffraction pattern of Form IV has characteristic peaks at the following 2θ positions: 7.95, 11.57, 12.08, 14.66, 16.04, 16.60, 18.12, 18.53, 18.96, 19.73, 23.32, and 25.24.

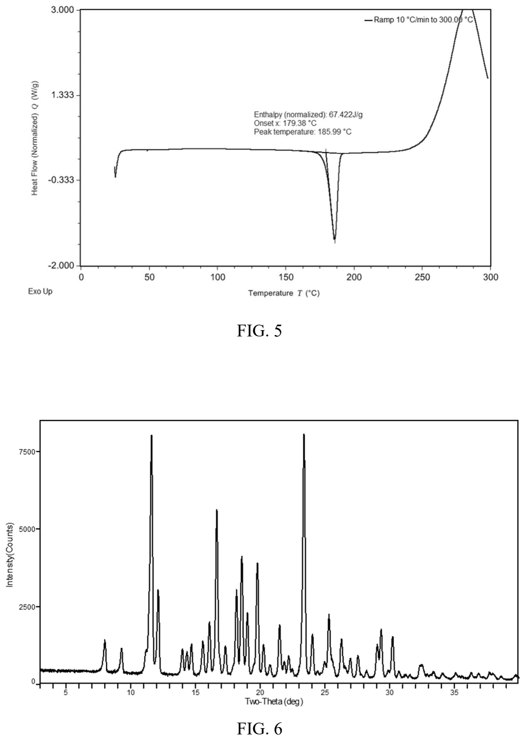

In some embodiments, the X-ray powder diffraction pattern of Form IV is shown in FIG. 6.

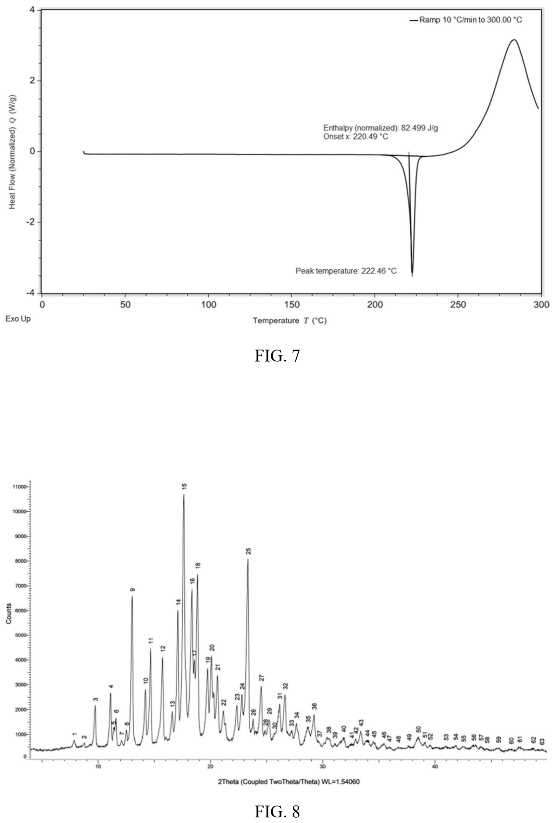

In some embodiments, Form IV melts at 224±3°C, and its DSC thermogram is shown in FIG. 7.

In another aspect of the present disclosure, the invention provides crystalline form V of 2-(3-(3-amino-4-(7H-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile. The X-ray powder diffraction pattern of Form V has characteristic peaks at the following 2θ positions: 9.73, 11.11, 13.03, 14.20, 14.66, 15.74, 17.10, 17.64, 18.35, 18.85, 20.09, and 23.33.

In some embodiments, the X-ray powder diffraction pattern of Form V is shown in FIG. 8.

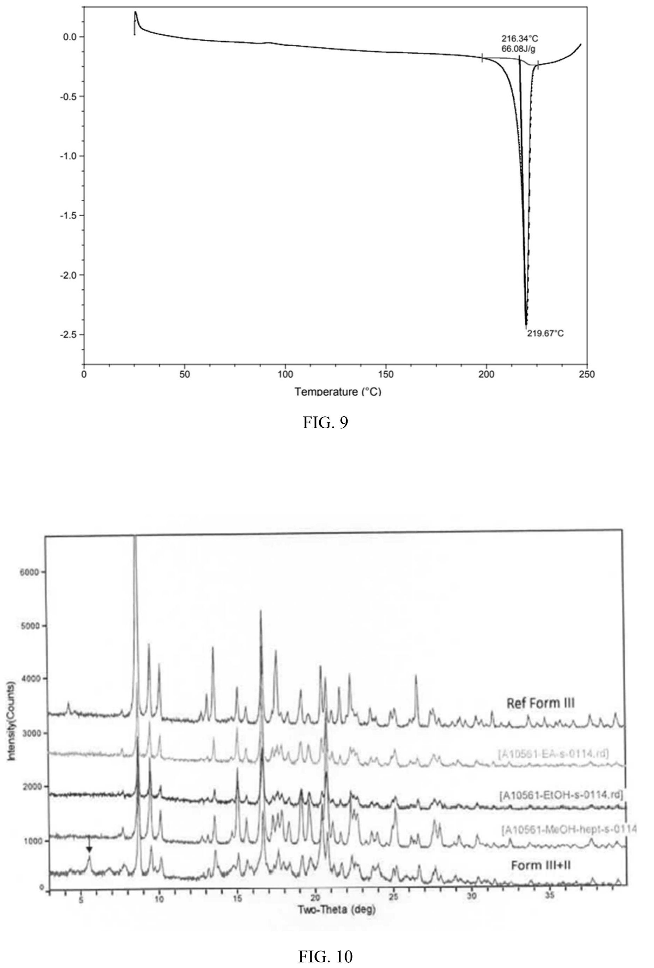

In some embodiments, Form V melts at 219±3°C, and its DSC thermogram is shown in FIG. 9.

In another aspect of the present disclosure, the invention provides a pharmaceutical composition comprising any of the aforementioned crystalline forms and a pharmaceutically acceptable carrier.

In another aspect of the present disclosure, the invention provides use of any of the aforementioned crystalline forms in the preparation of a JAK1 inhibitor.

In some embodiments, the JAK1 inhibitor is used to treat autoimmune-related diseases, including psoriasis, atopic dermatitis, vitiligo, pruritus, scleroderma, alopecia areata, total alopecia, diffuse alopecia, androgenetic alopecia, ankylosing spondylitis, psoriatic arthritis, rheumatoid arthritis, Crohn's disease, ulcerative colitis, systemic lupus erythematosus, and graft-versus-host disease.

### BRIEF DESCRIPTION OF THE DRAWINGS

FIG. 1 is an X-ray powder diffraction (XRD) pattern of Form I.
FIG. 2 is an X-ray powder diffraction (XRD) pattern of Form II.
FIG. 3 is a differential scanning calorimetry (DSC) thermogram of Form II.
FIG. 4 is an X-ray powder diffraction (XRD) pattern of Form III.
FIG. 5 is a differential scanning calorimetry (DSC) thermogram of Form III.
FIG. 6 is an X-ray powder diffraction (XRD) pattern of Form IV.
FIG. 7 is a differential scanning calorimetry (DSC) thermogram of Form IV.
FIG. 8 is an X-ray powder diffraction (XRD) pattern of Form V.
FIG. 9 is a differential scanning calorimetry (DSC) thermogram of Form V.
FIG. 10 is the XRD pattern of the competitive slurry of Form II/III.
FIG. 11 is the XRD pattern of the competitive slurry of Form III/IV/V.
FIG. 12 is a graph showing the stability of Form III at 25°C/60% RH for 7 days.
FIG. 13 is a graph showing the stability of Form IV at 25°C/60% RH for 7 days.
FIG. 14 is a graph showing the stability of Form V at 25°C/60% RH for 7 days.
FIG. 15 is a graph showing the crystalline stability of Form III/IV/V under accelerated and long-term conditions for 7 days.

### Detailed Description

The Form I of 2-(3-(3-amino-4-(7H-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile has an X-ray powder diffraction pattern as shown in FIG. 1, with a measurement error of ±0.2 degrees in 2θ, containing multiple absorption peaks between 0 and 50 degrees, as shown in Table 1.

**Table 1: d-values and 2θ angles of Form I**

| d-(Å) | 2θ angle | Relative strength (100%) |
|---|---|---|
| 15.813 | 5.584 | 100 |
| 12.636 | 6.990 | 24.1 |
| 11.041 | 8.001 | 30.7 |
| 8.920 | 9.908 | 28.3 |
| 6.354 | 13.926 | 26.9 |
| 5.900 | 15.003 | 31.9 |
| 4.327 | 20.509 | 82.6 |
| 3.954 | 22.469 | 19.3 |
| 3.680 | 24.167 | 19.3 |
| 3.437 | 25.899 | 18.3 |

The Form II of 2-(3-(3-amino-4-(*7H*-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile has an X-ray powder diffraction pattern as shown in FIG. 2, with a measurement error of ±0.2 degrees in 2θ, containing multiple absorption peaks between 0 and 50 degrees, as shown in Table 2.

**Table 2: d-values and 2θ angles of Form II**

| d-(Å) | 2θ angle | Relative strength (100%) |
|---|---|---|
| 15.914 | 5.548 | 57.1 |
| 12.897 | 6.848 | 21.1 |
| 11.203 | 7.885 | 33.8 |
| 9.041 | 9.775 | 16.9 |
| 6.420 | 13.781 | 29.7 |
| 5.571 | 15.894 | 24.3 |
| 5.407 | 16.379 | 19.8 |
| 5.248 | 16.880 | 30.4 |
| 4.911 | 18.047 | 14.1 |
| 4.541 | 19.532 | 11.3 |
| 4.344 | 20.424 | 100 |
| 3.705 | 23.995 | 26.0 |

The Form III of 2-(3-(3-amino-4-(*7H*-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile has an X-ray powder diffraction pattern as shown in FIG. 4, with a measurement error of ±0.2 degrees in 2θ, containing multiple absorption peaks between 0 and 50 degrees as shown in Table 3.

**Table 3: d-values and 2θ angles of Form III**

| d-(Å) | 2θ angle | Relative strength (100%) |
|---|---|---|
| 20.064 | 4.400 | 3.9 |
| 11.708 | 7.545 | 3.5 |
| 11.353 | 7.781 | 10.9 |
| 10.030 | 8.809 | 78.6 |
| 9.258 | 9.545 | 51.1 |
| 8.673 | 10.190 | 31.6 |
| 6.478 | 13.658 | 22.2 |
| 5.862 | 15.101 | 39.1 |
| 5.659 | 15.646 | 17.4 |
| 5.087 | 17.420 | 21.7 |
| 5.021 | 17.648 | 23.3 |
| 4.816 | 18.406 | 15.5 |
| 4.622 | 19.183 | 34.2 |
| 4.499 | 19.713 | 25.6 |
| 4.261 | 20.828 | 71.9 |
| 3.970 | 22.376 | 21.4 |

The Form IV of 2-(3-(3-amino-4-(*7H*-pyrrolo[2,3-*d*]pyrimidin-4-yl)-1H-pyrazol-1-yl)-1-(1-(3-fluoro-2-(trifluoromethyl)isonicotinyl)piperidin-4-yl)azetidin-3-yl)acetonitrile has an X-ray powder diffraction pattern as shown in FIG. 6, with a measurement error of ±0.2 degrees in 2θ, containing multiple absorption peaks between 0 and 50 degrees as shown in Table 4.

**Table 4: d-values and 2θ angles of Form IV**

| d-(Å) | 2θ angle | Relative strength (100%) |
|---|---|---|
| 11.075 | 7.976 | 20.0 |
| 9.525 | 9.277 | 10.1 |
| 7.630 | 11.588 | 100 |
| 7.305 | 12.105 | 30.1 |
| 6.340 | 13.957 | 9.6 |
| 6.183 | 14.311 | 10.2 |
| 6.036 | 14.662 | 12.6 |
| 5.699 | 15.535 | 11.0 |
| 5.518 | 16.047 | 21.8 |
| 5.337 | 16.597 | 60.1 |
| 5.132 | 17.263 | 8.4 |
| 4.888 | 18.131 | 29.9 |
| 4.784 | 18.529 | 34.9 |
| 4.675 | 18.966 | 17.6 |
| 4.493 | 19.743 | 42.0 |
| 3.815 | 23.297 | 71.8 |
| 3.525 | 25.241 | 18.8 |

The Form V of 2-(3-(3-amino-4-(*7H*-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile has an X-ray powder diffraction pattern as shown in FIG. 8, with a measurement error of ±0.2 degrees in 2θ, containing multiple absorption peaks between 0 and 50 degrees as shown in Table 5.

**Table 5: d-values and 2θ angles of Form V**

| d-(Å) | 2θ angle | Relative strength (100%) |
|---|---|---|
| 9.078 | 9.735 | 16.5 |
| 7.959 | 11.108 | 21.5 |
| 7.633 | 11.584 | 11.2 |
| 6.787 | 13.034 | 60.8 |
| 6.230 | 14.205 | 22.1 |
| 6.034 | 14.667 | 38.3 |
| 5.626 | 15.738 | 34.2 |
| 5.179 | 17.104 | 52.7 |
| 5.024 | 17.637 | 100 |
| 4.830 | 18.351 | 60.9 |
| 4.703 | 18.851 | 67.2 |
| 4.492 | 19.746 | 28.4 |
| 4.415 | 20.092 | 33.5 |
| 4.303 | 20.623 | 25.8 |
| 3.808 | 23.338 | 73.7 |
| 3.628 | 24.516 | 21.7 |

In the five tables above, the relative strength is defined as follows:

| Relative Strength | definition |
|---|---|
| 80-100 | VS (very strong) |
| 60-80 | S (strong) |
| 40-60 | M (medium) |
| 0-40 | W (weak) |

In this disclosure, the X-ray diffraction patterns were measured using the following method: instrument: Bruker D2 PHASER X-ray diffractometer; target: Cu: K-Alpha; wavelength λ = 1.54184 Å; tube voltage: 30 kV; tube current: 10 mA; scanning range: 3-40°; scanning speed: 0.2 s per step, 0.02° per step.

In this disclosure, differential scanning calorimetry (DSC) analysis was performed using the following method:
Instrument: Mettler DSC-1 Differential Scanning Calorimeter; Method: A sample (~5 mg) was heated in a DSC aluminum pan from 30°C to 300°C at a heating rate of 10°C/min.

It should be noted that in X-ray diffraction spectroscopy, the diffraction pattern obtained from a crystalline compound is often characteristic for a specific crystal form. The relative intensities of the bands (especially at low angles) may vary due to preferential orientation effects caused by differences in crystallization conditions, particle size, and other measurement conditions. Therefore, the relative intensities of the diffraction peaks are not characteristic for a specific crystal form. When determining whether a crystal form is identical to another crystal form, the relative positions of the peaks, rather than their relative intensities, should be considered more. Furthermore, for any given crystal form, the peak positions may vary slightly, as is well known in the art of crystallography. For example, peak positions can shift due to temperature fluctuations, sample movement, or instrument calibration during sample analysis, sometimes resulting in a measurement error of approximately ±0.2° in 2θ values. Therefore, this error should be taken into account when determining the structure of each crystal form. In XRD patterns, peak positions are typically expressed in 2θ angles or interplanar distances d. The two parameters can be converted using a simple equation: d = λ/2sinθ, where d represents the interplanar distance, λ represents the wavelength of the incident X-ray, and θ is the diffraction angle. For the same compound and the same crystal form, the peak positions of their XRD patterns are generally similar, but the relative intensity errors may be large. It should also be noted that in the identification of mixtures, some diffraction lines may be missing due to factors such as a decrease in content. In this case, it is not necessary to rely on all the bands observed in a high-purity sample; sometimes, even a single band may be characteristic for a given crystal.

It should be noted that DSC measures the transition temperature when a crystal absorbs or releases heat due to changes in its crystal structure or melting. For the same crystalline form of the same compound, the error in thermal transition temperatures and melting points in consecutive analyses is typically within approximately 5°C. When a compound is said to have a given DSC peak or melting point, this refers to the DSC peak or melting point ±5°C. DSC provides an auxiliary method for distinguishing different crystalline forms. Different crystal forms can be identified based on their distinct transition temperature characteristics.

The present disclosure also provides the synthesis of 2-(3-(3-amino-4-(7H-pyrrolo[2,3-*d*]pyrimidin-4-yl)-1H-pyrazol-1-yl)-1-(1-(3-fluoro-2-(trifluoromethyl)isonicotinyl)piperidin-4-yl)azetidin-3-yl)acetonitrile and the preparation of the Forms I, II, III, IV, and V. Furthermore, the preparation method of Form IV of the compound of formula I involved in the present disclosure is simple, the solvent is cheap and readily available, the crystallization conditions are mild, and it is suitable for industrial production.

### Examples

The following examples provide further non-limiting details of the technical solution of this disclosure. They should not be considered as limiting the scope of the present invention, but are merely exemplary and typical of the present invention.

### Example 1 2-(3-(3-amino-4-(7H-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile (I)

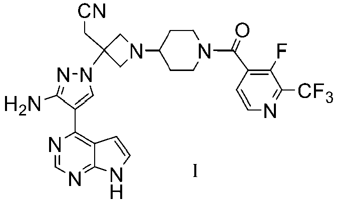

### Step A 2-{4-[7-{[2-(trimethylsilyl)ethoxy]methyl}-7H-pyrrolo[2,3-d]pyrimidin-4-yl]-1H-pyrazol-3-yl} isoindole-1,3-dione

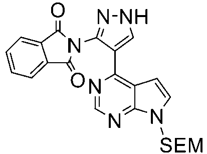

To a 3L of flask, add 1.5 L of toluene, 165 g (0.50 mol, 1.0 eq) of 4-(7-{[2-(trimethylsilyl)ethoxy]methyl}-7H-pyrrolo[2,3-d]pyrimidin-4-yl)-1H-pyrazol-3-amine, and 81.5 g (0.55 mol, 1.1 eq) of phthalic anhydride. Install a water separator and heat the mixture under reflux for 8 h. After heating, cool the reaction solution and filter. The resulting solid was dried at 55-60°C to a constant weight (191 g, yield 83.1%).

¹ H-NMR (400 MHz, DMSO- *d* ₆ ): δ 13.85 (s, 1H), 8.97 (d, 1H, *J* = 1.6 Hz), 8.25 (s, 1H), 8.14 - 7.91 (m, 4H), 7.80 (d, 1H, *J* = 3.7 Hz), 7.12 (d, 1H, *J* = 3.7 Hz), 5.61 (s, 2H), 3.52 (t, 2H, *J* = 8.0 Hz), 0.82 (t, 2H, *J* = 8.0 Hz), -0.09 (s, 9H); m/z =461.57[M+H] ⁺ .

### Step B: Benzyl 4-{3-(cyanomethyl)-3-[3-(1,3-phthaloyl-2-yl)-4-(7-((2-(trimethylsilyl)ethoxy)methyl)-7H-pyrrolo[2,3-d]pyrimidin-4-yl)-1H-pyrazol-1-yl]azetidin-1-yl}piperidine-1-carboxylate

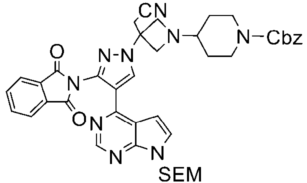

To a 2 L reaction flask, 138 g (0.30 mol, 1.0 eq) of 2- {4-[7-{[2-(trimethylsilyl)ethoxy]methyl}-7H-pyrrolo[2,3-*d*]pyrimidin-4-yl]-*1H*-*pyrazol-3-yl)isoindole-1,3-dione* , 9.13 g ( 0.06 mol, 0.2 eq) of 1,8-diazabicyclo[5.4.0]undec-7-ene, 450 mL of N,N-dimethylformamide, and 102.7 g (0.33 mol, 1.1 eq) of benzyl 4-(3-(cyanomethyl)azetidin-1-yl)piperidine-1-carboxylate were added, heated to 30-40°C, stirred and reacted for 3 h. The reaction was stopped, and 5 L of water and 1.5 L of ethyl acetate were added to the reaction solution for extraction. After ethyl acetate was concentrated, 600 mL of isopropanol was added, stirred and crystallized, and filtered. The resulting solid was dried at 55-60°C to obtain an off-white solid (200.8 g, yield 86.9%).

¹ H-NMR (400 MHz, DMSO- *d* ₆ ): δ 9.08 (s, 1H), 8.28 (s, 1H), 8.08 - 7.97 (m, 4H), 7.87 (d, 1H, *J =* 3.7 Hz), 7.47 - 7.31 (m, 5H), 7.20 (d, 1H, *J =* 3.7 Hz), 5.63 (s, 2H), 5.10 (s, 2H), 3.87 (t, 4H, *J =* 13.2 Hz), 3.65 (d, 4H, *J =* 8.0 Hz), 3.52 (t, 2H, *J =* 8.0 Hz), 3.35(s, 3H), 3.07 (s, 2H), 1.73 (d, 2H, *J* = 10.3 Hz,), 1.21 (dd, 2H), -0.09 (s, 9H, *J =* 24.3, 14.7 Hz); m/z=772.95[M+H] ⁺ .

### Step C: Benzyl 4-(3-(3-amino-4-(7H-pyrrolo[2,3-d]pyrimidin-4-yl)-1H-pyrazol-1-yl)-3-(cyanomethyl)azetidin-1-yl)piperidine-1-carboxylate

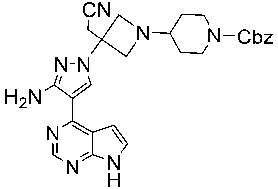

To a 2 L reaction flask, add 154 g (0.20 mol, 1.0 eq) of benzyl 4-{3-(cyanomethyl)-3-[3-(1,3-phthaloyl-2-yl)-4-(7-((2-(trimethylsilyl)ethoxy)methyl)-7H-pyrrolo[2,3-d]pyrimidin-4-yl)-1H-pyrazol-1-yl]azetidin-1-yl}piperidine-1-carboxylate and 700 mL of acetonitrile. Stir at room temperature and add dropwise 85.2 g (0.60 mol, 3.0 eq) of boron trifluoride in ether. After addition, raise the temperature to 40-50°C and stir for 5 h. Stop the reaction, concentrate the reaction solution, adjust the pH to 9-10 with saturated sodium carbonate solution, extract with 1 L of ethyl acetate, and concentrate to obtain an oily product.

Dissolve the oily product in 500 mL of ethanol, add 100 g (1.2 mol, 6.0 eq) of hydrazine hydrate (60% content), and heat to 70-80°C with stirring for 5 h. Stop the reaction, add 2 L of water to cool, and stir for crystallization. Filter, wash the filter cake with 300 mL of water, and dry at 55-60°C to obtain a light yellow solid (92.1 g, yield 90.0%).

¹ H-NMR (400 MHz, DMSO- *d* ₆ ): δ 12.08 (s, 1H), 8.69 (s, 1H), 8.53 (s, 1H), 7.57 (d, 1H, *J* =3.5Hz), 7.30-7.46 (m, 5H), 7.09 (d, 1H, *J* =3.6Hz), 6.34 (d, 2H, *J* =11.4Hz), 5.10 (s, 2H), 3.76-3.90(m, 2H), 3.70 (d, 2H, *J* =8.0Hz), 3.44-3.56 (m, 4H), 3.06 (s, 2H), 2.44 (dd, 1H, *J* =10.3, 6.9Hz), 1.69 (d, 2H, J=10.2Hz), 1.12-1.24 (m, 2H); m/z =512.59[M+H] ⁺ .

### Step D: Preparation of 2-(3-(3-amino-4-(7H-pyrrolo[2,3-d]pyrimidin-4-yl)-1H-pyrazol-1-yl)-1-(piperidin-4-yl)azacycl-3-yl)acetonitrile

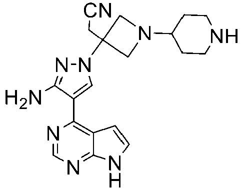

To a 5 L reaction flask, add 76.7 g (0.15 *mol , 1.0 eq) of benzyl* 4-(3-(3-amino-4-(7H -pyrrolo[2,3 -d]pyrimidin-4-yl)-1H-pyrazol-1-yl)-3-(cyanomethyl)azetidin-1-yl)piperidine-1-carboxylate and 760 mL of methanol/760 mL of tetrahydrofuran. Heat to 50-60°C and stir to dissolve. Add 7.6 g of Pd/C (0.015 mol, 0.1 eq) and allow reduction to proceed for 8 h. The reaction is then stopped, cooled, filtered, and concentrated. Add 500 mL of isopropyl ether, stir to crystallize, filter, and dry the filter cake at 55-60°C to obtain a light yellow solid (52.1 g, yield 92.0%).

¹ H-NMR (400 MHz, DMSO- *d* ₆ ): δ 8.69(s, 1H), 8.53(s, 1H), 7.57(d, 1H, *J* =3.5Hz), 7.09(d, 1H, *J* =3.6Hz), 6.34(d, 2H, *J* =11.2Hz), 3.66(d, 2H, *J* =8.1Hz), 3.41-3.53(m, 6H), 2.94(d, 2H, *J* =11.9Hz), 2.44(t, 2H, *J* =11.1Hz), 2.24(t, 1H, J=9.6Hz), 1.65(d, 2H, *J* =10.1Hz), 0.74-1.15(m, 2H); m/z =378.46[M+H] ⁺ .

### Step E: Preparation of 2-(3-(3-amino-4-(7H-pyrrolo[2,3-d]pyrimidin-4-yl)-1H-pyrazol-1-yl)-1-(1-(3-fluoro-2-(trifluoromethyl)isonicotinyl)piperidin-4-yl)azetidin-3-yl)acetonitrile (I)

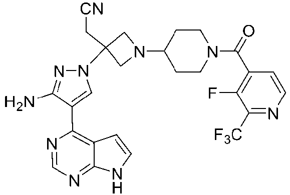

To a 2L reaction flask, add 50.0 g (0.13 mol, 1.0 eq) of 2-(3-(3-amino-4-(7H-pyrrolo[2,3-*d*]pyrimidin-4-yl)-1H-*pyrazol*-1-yl)-1-(piperidin-4-yl)azacycl-3-yl)acetonitrile, N,N-dimethylformamide (250 ml) / acetonitrile (250 ml), 30.7 g (0.16 mol, 1.2 eq) of 1-(3-dimethylaminopropyl)-3-ethylcarbodiimide hydrochloride, 18.4 g (0.16 mol, 1.2 eq) of N-hydroxysuccinimide, 39.5 g (0.39 mol, 3.0 eq) of triethylamine, and 29.3 g (0.14 mol, 1.1 eq) of 3-fluoro-2-trifluoromethyl-isonicotinic acid, and stir to dissolve. The mixture was heated to 30-40°C and allowed to react for 4 h. The reaction was stopped, and the mixture was extracted with 2.5 L of water and 1.5 L of ethyl acetate. Concentrated and 200 ml of ethanol was added for stirring to crystallize. The mixture was filtered, and the filter cake was dried at 55-60°C to obtain a light yellow solid (65.7 g, yield 87.25%).

¹ H NMR (400 MHz, DMSO- *d* ₆ ): δ 12.08(s, 1H), 8.69(q, 2H, *J* =4.8 Hz), 8.54(s, 1H), 7.94(t, 1H, J=4.7 Hz), 7.52-7.65(m, 1H), 7.09(dd, 1H, J=3.4, 1.5Hz), 6.35(s, 2H), 3.72 (d, 2H, *J* =7.6Hz), 3.42-3.58(m, 6H), 3.26- 3.33(m, 1H), 3.12 ( t, 1H, *J*=10.0 Hz ) , 2.58 ( s, 1H ) ,1.81(d, 1H, *J*=10.2 Hz),1.68(d, 1H, *J*=10.4 Hz), 1.29(dd, 2H, *J* =31.2, 8.9Hz); m/z =569.54 [M+H] ⁺ .

### Example 2 : Crystalline Form I of the Compound of Formula I

Take 2.0 g of the compound obtained in Example 1, add 6.0 ml of acetonitrile and stir to dissolve, stir and crystallize at room temperature from 20 to 30°C for 16 h, filter, rinse the filter cake with 2 ml of acetonitrile, and dry under reduced pressure at 50°C to obtain 1.62 g of the product.

The XRD pattern of Form I of the compound of Formula I is shown in FIG. 1.

### Example 3 : Crystalline Form II of the Compound of Formula I

### Method 1

5 mg of the crystal form I obtained in Example 2 was taken for variable temperature XRD, and it was found that the crystal form I was transformed into the crystal form II after desolvation.

### Method 2

Take 2.0 g of the compound obtained in Example 1, add a mixed solvent of 10 ml of acetonitrile and 10 ml of isopropyl acetate, heat to 70°C, dissolve and filter, cool to crystallize, filter, rinse the filter cake with 2 ml of the mixed solvent, and dry under reduced pressure at 50°C to obtain 1.03 g of the product.

The XRD pattern of Form II of the compound of Formula I is shown in FIG. 2.

The DSC thermogram of Form II of the compound of Formula I is shown in FIG. 3.

### Example 4 : Crystalline Form III of the Compound of Formula I

1.8 g of Form I of the compound obtained in Example 2 was suspended in 7 ml of ethanol and stirred at room temperature overnight. 3 ml of methyl tert-butyl ether was then added as an antisolvent and stirred for several hours. The solid was collected by filtration and dried in vacuo at 40°C for 4 hours to obtain 1.4 g of the product .

The XRD pattern of Form III of the compound of Formula I is shown in FIG. 4.

The DSC thermogram of Form III of the compound of Formula I is shown in FIG. 5.

### Example 5 : Crystalline Form IV of the Compound of Formula I

2.0 g of the compound obtained in Example 2 was added to 20 ml of water, heated to 60-70°C, stirred and crystallized for 6 hours, filtered, the filter cake was rinsed with 10 ml of water, and dried under reduced pressure at 50 °C to obtain 1.80 g of the product.

The XRD pattern of Form IV of the compound of Formula I is shown in FIG. 6.

The DSC thermogram of Form IV of the compound of Formula I is shown in FIG. 7.

### Example 6 : Crystalline Form V of the Compound of Formula I

2.0 g of the compound obtained in Example 1 was added to 6 ml of ethanol at room temperature (20-30°C), stirred and crystallized for 16 hours, and filtered. The filter cake was rinsed with 2 ml of solvent and dried under reduced pressure at 50°C to obtain 1.46 g of the product.

The XRD pattern of Form V of the compound of Formula I is shown in FIG. 8.

The DSC thermogram of Form V of the compound of Formula I is shown in FIG. 9.

### Example 7: Evaluation of the Crystal Forms

Each crystalline form of the compound of formula I was evaluated, including competitive slurry test, stability and solubility studies.

### 1. Competitive slurry test

The relative stability of Form II and Form III was investigated by competitive slurry test. Approximately 5 mg of Form II and Form III were weighed, mixed, and then added in 1/1 ratio of methanol/n-heptane, ethanol and ethyl acetate, respectively, to prepare a suspension. The suspension was stirred at room temperature for several hours, then centrifuged and the solid was analyzed by XRD. The results showed that the solid transformed into Form III, indicating that Form III is more stable than Form II.

The competitive slurry XRD patterns of Form II and Form III are shown in FIG. 10.

Competitive slurry studies were conducted to investigate the relative stability of Forms III, IV, and V. 5 mg of Forms III, IV, and V were weighed and mixed uniformly. Suspensions were prepared respectively in ethanol (80°C), ethanol/water (1/1) (80°C), water (60°C), water (25°C), acetonitrile (70°C), and acetonitrile/water (1/1) (70°C). The suspensions were stirred for 12 hours, and the solids were centrifuged and analyzed by XRD.

**Table 6: Competitive slurry results of Forms III, IV and V**

| Serial number | raw material | solvent | 12h-crystal form |
|---|---|---|---|
| 1 | | Ethanol (80°C) | IV+V |
| 2 | | Ethanol/water (1/1) (80°C) | IV |
| 3 | III:IV:V=1:1:1 | Water (60°C) | IV+V |
| 4 | | Water (25°C) | III+IV+V |
| 5 | | Acetonitrile (70°C) | IV |
| 6 | | Acetonitrile/water (1/1) (70°C) | IV |

The results showed that Form III and Form V both transformed into Form IV in the ethanol/water (1/1) (80°C), acetonitrile (70°C), and acetonitrile/water (70°C) systems. Therefore, Form IV is the most stable of the five forms.

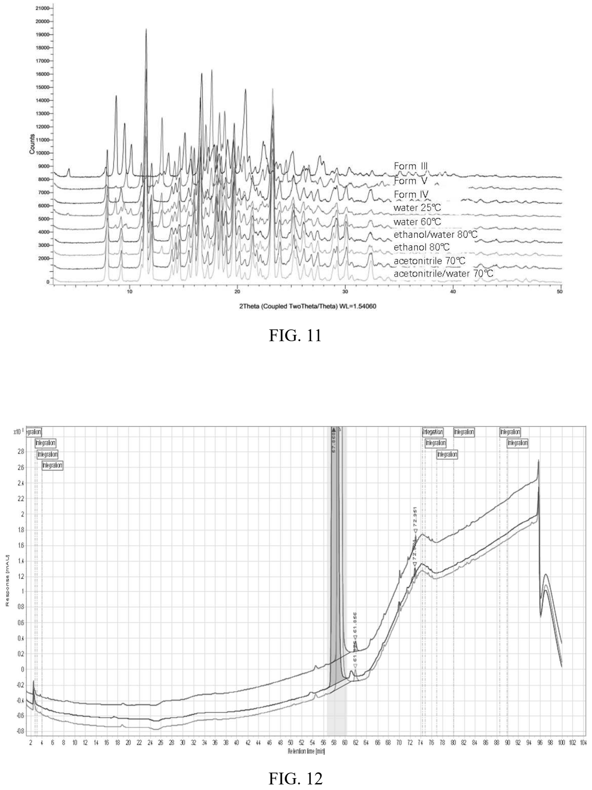

The competitive slurry XRD patterns of Forms III, IV, and V are shown in FIG. 11.

### 2. Stability Investigation

The analysis method is as follows:
Octadecylsilane bonded silica gel was used as the filler (Kromasil C ₁₈ 4.6 mm × 250 mm, 5 µm); 0.01 mol/L potassium dihydrogen phosphate solution (pH was adjusted to 3.0 with phosphoric acid) was used as mobile phase A, and methanol was used as mobile phase B. Gradient elution was performed according to the table below; the detection wavelength was 220 nm; the column temperature was 40°C; the flow rate was 1.0 ml/min; the injection volume was 10 µl, and the HPLC purity was calculated by the area normalization method.

| Time (minutes) | Mobile phase A (%) | Mobile phase B (%) |
|---|---|---|
| 0 | 80 | 20 |
| 20 | 80 | 20 |
| 22 | 70 | 30 |
| 33 | 70 | 30 |
| 60 | 52 | 48 |
| 70 | 24 | 76 |
| 92 | 10 | 90 |
| 92.1 | 80 | 20 |
| 100 | 80 | 20 |

Appropriate amounts of Form III, IV, and V were weighed in sample bottles and stored uncovered at 80°C, 40°C/75% RH, and 25°C/60% RH for 7 days. Samples were collected at 0, 1, 3, and 7 days for HPLC and XRD analysis.

**Table 7 Stability test results of Form III, IV and V**

| Condition | | Form III | | Form IV | | Form V | |
|---|---|---|---|---|---|---|---|
| | | 220nm | Crystal form | 220nm | Crystal form | 220nm | Crystal form |
| 0 days | | 99.83% | | 99.57% | | 98.02% | |
| | 1 day | 99.90% | | 99.57% | | 98.05% | |
| 80°C | 3 days | 99.89% | | 99.60% | | 98.03% | |
| | 7 days | 99.91% | III | 99.57% | IV | 98.02% | V |
| 40°C/75%RH | 3 days | 99.89% | | 99.53% | | 98.02% | |
| | 7 days | 99.90% | | 99.63% | | 98.01% | |
| 25°C/60%RH | 3 days | 99.90% | | 99.59% | | 98.10% | |
| | 7 days | 99.95% | | 99.63% | | 97.87% | |

The results showed that the chemical stability of Form III, Form IV and Form V was relatively stable under three conditions of 80°C, 40°C/75%RH and 25°C/60%RH for 7 days, and the crystal forms did not change.

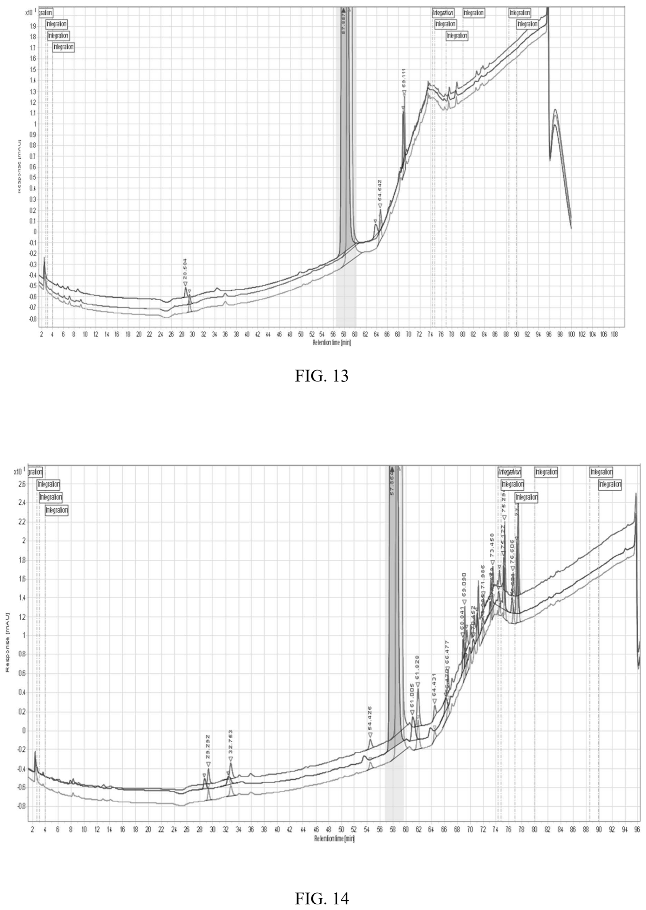

The chemical stability profiles of Forms III, IV, and V are shown in FIG. 12, FIG. 13, and FIG. 14.

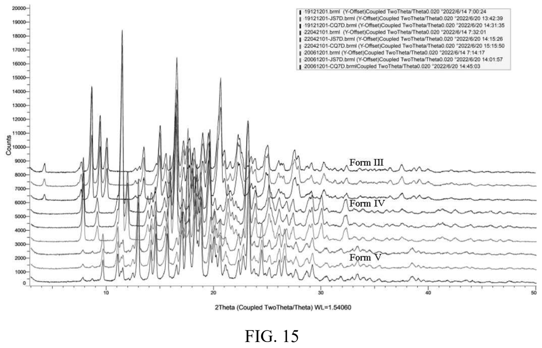

The crystal stability profiles of Forms III, IV, and V are shown in FIG. 15.

### 3. Solubility Determination

Appropriate amounts of Forms III, IV, and V were weighed and added to buffer solutions with pH 4.5 and 6.8 to prepare a suspension at 5 mg/mL. The resulting suspension was stirred at 37°C, and samples were collected and analyzed at 5, 30, 60, and 240 minutes. Use a syringe to draw approximate 1 mL of the suspension, filter it through a membrane filter, and dilute it several times before testing the resulting filtrate with HPLC, using the area normalization method to determine the solubility.

**Table 8 Tests of different crystal forms at various pH values**

| Time (min) | PH=4.5 (mg/ml) | | | pH=6.8 (mg/ml) | | |
|---|---|---|---|---|---|---|
| | Form III | Form IV | Form V | Form III | Form IV | Form V |
| 5 | 0.0413 | 0.0471 | 0.0112 | 0.0112 | 0.0247 | 0.0076 |
| 30 | 0.0601 | 0.0656 | 0.0258 | 0.0299 | 0.0372 | 0.0133 |
| 60 | 0.0655 | 0.0700 | 0.0289 | 0.0332 | 0.0402 | 0.0160 |
| 240 | 0.0840 | 0.0734 | 0.0356 | 0.0415 | 0.0402 | 0.0190 |

The results showed that in pH = 4.5 (postprandial gastric juice), the solubility of Form IV at 240 min was comparable to that of Form III, which was superior to that of Form V and twice that of Form V. In pH = 6.8 (simulated intestinal fluid), the solubility of Form IV at 240 min was comparable to that of Form III and twice that of Form V. Therefore, Form IV exhibited good solubility.

### Example 8: Biological Activity Test

### 1. Enzymatic activity (IC₅₀) test

A JAK1/2/3 kinase activity detection platform was established using the Lance Ultra principle to measure the activity of the compounds, while the known Itacitinib was also measured as a control. In the detection plate, the enzyme, Ulight-labeled peptide substrate, ATP, and the test compound were mixed and incubated. After the reaction, EDTA was added to terminate the reaction, and Eu-labeled antibodies were added for detection. The detection plate was analyzed using PE's Envision, and the analysis mode was TR-FRET. The data were represented by the readings of the fluorescence signal at 665nm and 615nm, respectively. A high ratio of 665nm/615nm indicates high enzyme activity, while a low ratio of 665nm/615nm indicates that the enzyme activity is inhibited.

Reagents: Kinases (JAK1/2/3), substrates (ULight-JAK-1 peptide and ATP), detection reagents (Eu-W1024 Anti-phosphotyrosine and EDTA). Instruments: Echo, Envision.

Dissolve the test compound in DMSO to a 10 mM solution and store in a nitrogen atmosphere for long-term storage. Dilute 10 µL of the 10 mM test compound solution to a 1 mM working solution. Use an Echo to make three-fold dilutions, for a total of 11 concentrations, with the final reaction concentration ranging from 10 µM to 0.17 nM. Use an electronic pipette to add 5 µL of the mixture of enzyme and peptide substrate to the assay plate. Centrifuge the plate and incubate it at room temperature (23 ° C) for 15 minutes. Use an electronic pipette to add 5 µL of kinase buffer containing ATP to the plate. Centrifuge the plate, seal it with aluminum foil, and incubate it at room temperature (23 ° C) for 90 minutes. Terminate the reaction, add detection reagent to the plate using an electronic pipette, centrifuge the plate, seal it with aluminum foil, and incubate it at room temperature (23 ° C) for half an hour. Measure the signal of the reaction plate with the Envision instrument. The results show that the IC₅₀ values of the compound of formula I (amorphous form) and the control Itacitinib or Barcitinib are all less than 10 nM.

### 2. Determination of bioavailability in adult SD rats

Healthy adult female Sprague-Dawley rats were obtained from Beijing Weitong Lihua Laboratory Animal Technology Co., Ltd. The study was conducted in two phases: first, animals received a single intravenous injection of 1 mg/kg; second, one week later, the same group received a single oral gavage of the suspension at a dose of 10 mg/kg. Animals receiving oral gavage were fasted overnight and from 10 hours before to 4 hours after administration. Animals in the intravenous group were not restricted to food. Blood samples were collected at 0.083, 0.25, 0.5, 1, 2, 4, 6, 8, and 24 hours after administration for the intravenous group and at 0.25, 0.5, 1, 2, 4, 8, and 24 hours after administration for the oral group. After isoflurane anesthesia by using a small animal anesthesia machine, 0.4 ml of whole blood was obtained from the retinal venous plexus. The sample was centrifuged at 4000 rpm for 20 minutes at 4°C. Plasma was separated and placed in labeled EP tubes. Plasma samples were immediately stored in an ultra-low temperature freezer until analysis. Plasma samples should be stored in a refrigerator at -70°C before testing. Plasma samples were extracted using protein precipitation, and the extracts were analyzed by LC/MS/MS.

| PK parameters | unit | Compound of formula I (Form IV) | |
|---|---|---|---|
| | | IV (1 mg/kg) | PO (10 mg/kg) |
| t _{1/2} | h | 0.61 | 1.90 |
| Tmax | h | -- | 0.5 |
| C ₘₐₓ | ng/mL | -- | 475 |
| Cl _{s,app} | mL/min/kg | 37.7 | -- |
| VZ | L/kg | 1.50 | -- |
| AUC _{0-inf} | h*ng/mL | 456 | 783 |
| Bioavailability | h*ng/mL | -- | 17% |

It can be seen from the above PK (IV and PO) data that the PO bioavailability of the compound of Formula I (Form IV) reaches 17%.

### 3. Pharmacokinetic (PK) determination in adult Beagle dogs

Healthy adult male Beagle dogs were obtained from Beijing Marshall Biotechnology Co., Ltd. Three groups were divided into this study and administered via oral gavage at a dose of 19.5 mg/kg. Animals receiving oral gavage were fasted overnight and from 10 hours before to 4 hours after administration. Blood samples were collected 15 minutes, 30 minutes, 45 minutes, 1 hour, 1.5 hours, 3 hours, 4 hours, 6 hours, 8 hours, and 24 hours after gavage. Blood samples (1 mL) were collected from the forelimb vein or other suitable venous sampling methods and placed in labeled EDTA-K2 anticoagulant tubes. After gentle inversion to thoroughly mix the anticoagulant with the blood, the tubes were immediately placed on wet ice. Plasma was separated by centrifugation within 1 hour of blood collection in the condition of 4°C, 2200g, and 10 minutes. The separated plasma was placed in labeled EP tubes. Plasma samples were immediately stored in an ultra-low temperature freezer until analysis. Plasma samples should be stored in a refrigerator at -70°C before testing. Plasma samples were extracted using protein precipitation, and the extracts were analyzed by LC/MS/MS.

| PK parameters | unit | **Form III** | **Form IV** | **Form V** |
|---|---|---|---|---|
| t _{1/2} | h | 2.45 | 2.63 | 2.86 |
| Tmax | h | 1.00 | 1.00 | 1.00 |
| C ₘₐₓ | ng/mL | 4057 | 6197 | 2440 |
| AUC ₀₋ₜ | h*ng/mL | 13174 | 27750 | 8354 |
| AUC _{0-inf} | h*ng/mL | 13248 | 27789 | 8431 |

Through comprehensive analysis of the PK data and other data of this disclosure, it can be seen that the crystalline form IV of the compound of Formula I exhibits better pharmacokinetic properties after PO administration.

From the analysis in the aspects of bioactivity, bioavailability, stability, solubility and pharmacokinetic properties, it can be seen that the Form IV has better drugability.

## Claims

1. A crystalline form of 2-(3-(3-amino-4-(*7H*-pyrrolo[2,3- d]pyrimidin-4-yl)-1H-pyrazol-1-yl)-1-(1-(3-fluoro-2-(trifluoromethyl)isonicotinyl)piperidin-4-yl)azetidin-3-yl)acetonitrile, wherein the crystalline form is designated as Form I and has an X-ray powder diffraction pattern (XRPD) comprising characteristic diffraction peaks at the following 2 ^{θ} positions: 5.17, 5.55, 6.30, 8.67, 10.34, 15.96, 19.06, 19.75, 20.39, and 21.42.

2. The crystalline form I according to claim 1, wherein the X-ray powder diffraction pattern is shown in FIG. 1.

3. A crystalline form of 2-(3-(3-amino-4-(*7H*-pyrrolo[2,3- d]pyrimidin-4-yl)-1H-pyrazol-1-yl)-1-(1-(3-fluoro-2-(trifluoromethyl)isonicotinyl)piperidin-4-yl)azetidin-3-yl)acetonitrile, wherein the crystalline form is designated as Form II and has an X-ray powder diffraction pattern (XRPD) comprising characteristic diffraction peaks at the following 2θ positions: 5.55, 6.85, 7.89, 9.78, 13.78, 15.89, 16.38, 16.88, 18.05, 20.42, and 24.00.

4. The crystalline form II according to claim 3, wherein the X-ray powder diffraction pattern is shown in FIG. 2.

5. A crystalline form of 2-(3-(3-amino-4-(*7H*-pyrrolo[2,3- d]pyrimidin-4-yl)-1H-pyrazol-1-yl)-1-(1-(3-fluoro-2-(trifluoromethyl)isonicotinyl)piperidin-4-yl)azetidin-3-yl)acetonitrile, wherein the crystalline form is designated as Form III and has an X-ray powder diffraction pattern (XRPD) comprising characteristic diffraction peaks at the following 2θ positions: 8.76 , 9.53, 10.17, 15.11, 16.70, 19.18, 20.49, 20.79, 22.33, and 25.18.

6. The crystalline form III according to claim 5, wherein the X-ray powder diffraction pattern is shown in FIG. 4.

7. A crystalline form of 2-(3-(3-amino-4-(*7H*-pyrrolo[2,3- *d*]pyrimidin-4-yl)-1H-pyrazol-1-yl)-1-(1-(3-fluoro-2-(trifluoromethyl)isonicotinyl)piperidin-4-yl)azetidin-3-yl)acetonitrile, wherein the crystalline form is designated as Form IV and has an X-ray powder diffraction pattern (XRPD) comprising characteristic diffraction peaks at the following 2θ positions: 7.95, 11.57, 12.08, 14.66, 16.04, 16.60, 18.12, 18.53, 18.96, 19.73, 23.32, and 25.24.

8. The crystalline form IV according to claim 7, wherein the X-ray powder diffraction pattern is shown in FIG. 6.

9. The crystalline form IV according to claim 7 or 8, wherein the crystalline form melts at 224±3°C, as shown in FIG. 7.

10. A crystalline form of 2-(3-(3-amino-4-(*7H*-pyrrolo[2,3- *d*]pyrimidin-4-yl)-1H-pyrazol-1-yl)-1-(1-(3-fluoro-2-(trifluoromethyl)isonicotinyl)piperidin-4-yl)azetidin-3-yl)acetonitrile, wherein the crystalline form is designated as Form V and has an X-ray powder diffraction pattern (XRPD) comprising characteristic diffraction peaks at the following 2θ positions: 9.73, 11.11, 13.03, 14.20, 14.66, 15.74, 17.10, 17.64, 18.35, 18.85, 20.09, and 23.33 .

11. The crystalline form V according to claim 10, wherein the X-ray powder diffraction pattern is shown in FIG. 8.

12. The crystalline form V according to claim 10 or 11, wherein the crystalline form melts at 219±3°C, as shown in FIG. 9.

13. A pharmaceutical composition comprising the crystalline form of any of the preceding claims.

14. Use of the crystalline form of any of the preceding claims in the preparation of a JAK1 inhibitor.

15. The use according to claim 14, wherein the JAK1 inhibitor is used to treat autoimmune-related diseases, including psoriasis, atopic dermatitis, vitiligo, pruritus, scleroderma, alopecia areata, total alopecia, diffuse alopecia, androgenetic alopecia, ankylosing spondylitis, psoriatic arthritis, rheumatoid arthritis, Crohn's disease, ulcerative colitis, systemic lupus erythematosus, and graft-versus-host disease.
